# EUROPEAN PATENT SPECIFICATION

(11) **EP 2 593 028 B1**
(45) Date of publication and mention of the grant of the patent: **16.08.2017**
(21) Application number: 11754809.9
(22) Date of filing: 01.09.2011
(51) Int. Cl.: A61B 18/02

(54) **CRYOSURGICAL INSTRUMENT FOR TREATING LARGE VOLUME OF TISSUE**
KRYOCHIRURGISCHES INSTRUMENT ZUR BEHANDLUNG VON GROSSEN GEWEBEMENGEN
INSTRUMENT CRYOCHIRURGICAL POUR TRAITER UN GRAND VOLUME DE TISSU

(30) Priority: 15.09.2010 US 382953 P
(43) Date of publication of application: 22.05.2013
(73) Proprietor: Icecure Medical Ltd., 38900 Caesarea (IL)
(72) Inventor: BERZAK, Nir, 53271 Givataim (IL); SHARON, Simon, 30805 Ma'ayan Zvi (IL); HILLELI, Ron, 30900 Zichron Yaacov (IL)
(74) Representative: Hanna Moore + Curley
(86) International application number: PCT/US2011/050214
(87) International publication number: WO 2012/036914

(56) References cited:
- US-A- 4 917 106
- US-A- 5 800 432
- US-A1- 2004 002 699
- US-A1- 2006 122 590
- US-A1- 2007 167 938
- US-A1- 2008 051 776

## Description

### BACKGROUND OF THE INVENTION

### 1. Technical Field

Embodiments of the present invention relate generally to cryosurgical instruments such as cryoprobes and, more particularly, to cryosurgical instruments suitable for the ablation of a large volume of tissue.

### 2. Description of related art

Cryosurgical ablation of a large volume of tissue typically involves placement of several (i.e., multiple) cryosurgical instruments within a defined volume to cause continuous ablation of the tissue in that volume (see US2006/0122590 for a gas-cooled cryoprobe).

Two principles are used to assist the physician to obtain the desired relationship between the cryosurgical instruments: some type of guide, such as an external template, or guiding sleeves; and clustering the cryosurgical instruments for insertion to the defined volume, typically simultaneously. The cryosurgical instrument penetrates the tissue along its main axes. The templates or the guiding sleeves can bend the flexible cryosurgical instrument and direct it in relationship to the other inserted cryosurgical instruments. However, the instrument may only be bent at a narrow angle, in order to maintain the integrity of the instrument.

The proper positioning of the cryosurgical instruments in relation to each other depends on the flexibility of the cryosurgical instruments, the diameter of the guide (and hence the degree of "play" or potential inaccuracy in guiding the instruments), and the skill of the physician. An instrument with greater flexibility may be more easily bent within the template or the sleeve, but once it is outside this guide, the instrument may bend further and so may not maintain the desired position. The diameter of the guiding element is always larger than the diameter of the cryosurgical instrument and therefore, further introduces error. Finally it is the judgment of the physician through the information received by the imaging tool, such as ultrasound imaging, CT or MRI, to determine whether the position of the cryosurgical instruments is adequate in three-dimensional space. Since the cryoablation volume induced by a single individual instrument is small in size, the relationship between the cryosurgical instruments is crucial to the success of the treatment. As a result, a physician may frequently insert more cryosurgical instruments than necessary to treat the required volume.

An additional challenge in the placement of several cryosurgical instruments in the selected tissue is caused by the deformability of human tissue, and the need for fixation of the organ within the body. To solve this problem two approaches are used: (1) cooling the first instrument to a temperature that effectively sticks the instrument to the tissue (creating contact surface of -20°C or lower); or (2) holding the organ in place, using a mechanical anchor such as cork-screw element, while inserting several cryosurgical instruments into it.

The use of corkscrew (i.e., helical) type elements as mechanical anchors for various cryosurgical purposes is known (see, e.g., U.S. Patent and Patent Publication Nos. 6,343,605, 6,004,269, 7,567,838, 2006/0253080, 2009/0292279 and 2010/0015196) or as a grasper (see. e.g., U.S. Patent Publication No. 2008/0294179).

The use corkscrew type elements to attach other components to each other is also known (see, e.g., U.S. Patent Nos. 4917106 and 5,195,540 and U.S. Patent Publication No. 2006/0259050). Similarly, a thermocouple has been attached to the cork screw element as a sensor during surgery (see, e.g., U.S. Patent Nos. 5,800,432, 5,688,266, 5,688,267, 6,053,912, and 5,735,846.

A corkscrew type element has been also used to transmit electrical signals (see, e.g., U.S. Patent No. 5,6269,272 and U.S. Patent Publication No. 2005/0101984), and as a RF electrode (see, e.g., U.S. Patent Publication 2004/0147917).

A corkscrew element has also been used as a biopsy tissue sampler (see, e.g., U.S. Patent Nos. 4,682,606 and 6,142,957 and U.S. Patent Publication No. 2004/0147917) and as a lesion marker (U.S. Patent No. 5,195,540) or as a vertebral support (U.S. Patent Publication No. 2008/0140203).

### BRIEF SUMMARY

The background art does not provide, however, a helical element, introduced by rotation, suitable for cryosurgical applications, in which the element transmits cryogens and ablates a large volume of tissue without the placement of several (i.e., multiple) cryosurgical instruments within a defined volume to cause continuous ablation of the tissue in that volume.

One aspect of the present invention provides a cryosurgical instrument that is selectively positioned in a patient by rotation, including: a manipulation section that permits a user to rotate the instrument; a cryogen supply portion; and a positioning section having a sharp tip at an end thereof and a helical configuration, the positioning section configured to receive cryogen from the cryogen supply portion and to permit the received cryogen to cool the positioning section.

A further aspect of the present invention provides a cryosurgical instrument having a substantially smooth, temperature conducting outer surface with an inner side, including: a sharp tip at an end to facilitate penetration into tissue; one or more cryogen supply lines that supply cryogen to the instrument; a manipulation section for positioning the instrument, at least a portion of the manipulation section extending along and defining a longitudinal axis of the instrument, the manipulating section having insulation along at least a portion of a length thereof, and a return fluid sleeve to permit exhaust of expanded fluid from the instrument; a cooling section connected to the manipulation section, extending from the manipulation section to the tip, and having a helical configuration spiraling around the longitudinal axis, and a heat exchanger in fluid communication with the one or more cryogen supply lines, surrounding the one or more cryogen supply lines in the cooling section, and delivering received cryogen to a port at a distal end thereof proximal to the tip, the heat exchanger including a plurality of channels that are circumferentially disposed along the inner side of the outer surface and that interconnect the one or more supply lines to the a return gas sleeve, the heat exchanger permitting cryogen from the one or more supply lines to cool the cooling section; and an expanded fluid return pathway surrounding the one or more cryogen supply lines and bounded by the one or more cryogen supply lines and the inner side of the outer surface of the instrument so that gas in the pathway is in thermal communication with the inner side.

Another aspect of the present invention provides a cryosurgical instrument having a substantially smooth, temperature conducting outer surface with an inner side, including: a sharp tip at an end to facilitate penetration into tissue; a manipulation section for positioning the instrument, at least a portion of the manipulation section extending along and defining a longitudinal axis of the instrument, the manipulating section having insulation along at least a portion of its length; a cooling section connected to the manipulation section, extending from the manipulation section to the tip, and having a helical configuration spiraling around the longitudinal axis; one or more cryogen supply lines that supply cryogen to the instrument, each supply line delivering cryogen to one or more ports along a portion of a length thereof in the cooling section, at least one of the supply lines having a port at a distal end thereof proximal to the tip; and a return fluid sleeve to permit exhaust of expanded gas from the instrument, the sleeve surrounding the one or more cryogen supply lines and bounded by the one or more cryogen supply lines and the inner side of the outer surface of the instrument so that fluid in the sleeve is in thermal communication with the inner side, the sleeve permitting cryogen from the one or more supply lines to cool the cooling section.

Still another aspect of the present invention provides a cryosurgical instrument having a substantially smooth, temperature conducting outer surface with an inner side, including: a sharp tip at an end to facilitate penetration into tissue; a cryogen supply line that delivers cryogen to a port at an end proximal to the tip; a manipulation section for positioning the instrument, at least a portion of the manipulation section extending along and defining a longitudinal axis of the instrument, the manipulating section having insulation along at least a portion of a length thereof, and a return fluid sleeve to permit exhaust of expanded gas from the instrument; a cooling section connected to the manipulation section, extending from the manipulation section to the tip, and having a helical configuration spiraling around the longitudinal axis, and a barrier that is disposed only in the cooling section and spirals about the cryogen supply line, the barrier yielding a spiraling channel that is circumferentially disposed along the inner side of the outer surface, that is bounded by the cryogen supply line and the inner side of the outer surface of the instrument, and that interconnects the supply line to the a return gas sleeve, the barrier permitting cryogen exiting the port to cool the cooling section.

Yet another aspect of the present invention provides a cryosurgical instrument having a substantially smooth, temperature conducting outer surface with an inner side, including: a sharp tip at an end to facilitate penetration into tissue; a cryogen supply line that delivers cryogen to a port at an end proximal to the tip; a manipulation section for positioning the instrument, at least a portion of the manipulation section extending along and defining a longitudinal axis of the instrument, the manipulating section having insulation along at least a portion of a length thereof, and a return fluid sleeve to permit exhaust of expanded gas from the instrument; a cooling section connected to the manipulation section, extending from the manipulation section to the tip, and having a helical configuration spiraling around the longitudinal axis, and a core that is disposed only in the cooling section and that extends from the manipulating section to substantially near the tip. The cryogen supply line spirals around the core. The spiraling of the cryogen supply line yields a spiraling channel that is circumferentially disposed along the inner side of the outer surface, that is bounded by the cryogen supply line and the inner side of the outer surface of the instrument, and that interconnects the supply line to the a return fluid sleeve, the spiraling channel permitting cryogen exiting the port to cool the cooling section.

Optionally, the cryosurgical instrument features a single tip and in operation, is used singly to ablate the large volume of tissue, without requiring the insertion of multiple cryosurgical instruments.

This Summary is provided to introduce a selection of concepts in a simplified form that are further described below in the Detailed Description. This Summary is neither intended to identify key features or essential features of the claimed subject matter, nor should it be used to limit the scope of the claimed subject matter. Furthermore, the claimed subject matter is not limited to implementations that solve any disadvantage noted in any part of this application.

These, additional, and/or other aspects and/or advantages of the present invention are: set forth in the detailed description which follows; possibly inferable from the detailed description; and/or learnable by practice of the present invention.

### BRIEF DESCRIPTION OF THE DRAWINGS

The present invention will be more readily understood from the detailed description of embodiments thereof made in conjunction with the accompanying drawings of which:
FIG. 1a is a partial cut-away view of a cryosurgical instrument that is consistent with an embodiment of the present invention;
FIG. 1b is a cross-sectional view of the cryosurgical instrument of FIG. 1a taken along line A-A of FIG. 1a;
FIG. 2a is a partial cut-away view of a cryosurgical instrument that is consistent with an embodiment of the present invention;
FIG. 2b is a cross-sectional view of the cryosurgical instrument of FIG. 1a taken along line A-A of FIG. 1a;
FIG. 3 is a side view of a cryosurgical instrument that is consistent with an embodiment of the present invention;
FIG. 4 is a side view of a cryosurgical instrument that is consistent with an embodiment of the present invention;
FIG. 5a is a partial cut-away, side view of a cryosurgical instrument that is consistent with an embodiment of the present invention;
FIG. 5b is a perspective view of a heat exchanger of the cryosurgical instrument of FIG. 5a;
FIG. 5c is a cross-sectional view of the cryosurgical instrument of FIG. 5a taken along line A-A of FIG. 5a;
FIG. 6a is a partial cut-away, side view of a cryosurgical instrument that is consistent with an embodiment of the present invention;
FIG. 6b is a cross-sectional view of the cryosurgical instrument of FIG. 6a taken along line A-A of FIG. 6a;
FIG. 7 is a partial cut-away, side view of a cryosurgical instrument that is consistent with an embodiment of the present invention;
FIG. 8 is a partial cut-away, side view of a cryosurgical instrument that is consistent with an embodiment of the present invention;
FIG. 9 is a partial cut-away, side view of a cryosurgical instrument that is consistent with an embodiment of the present invention;
FIGS. 10a and 10b respectively illustrate a system that is consistent with an embodiment of the present invention in a retracted and a protracted state; and
FIG. 11 is a side view of a cryosurgical instrument that is consistent with an embodiment of the present invention.

### DETAILED DESCRIPTION

Reference will now be made in detail to embodiments of the present invention, examples of which are illustrated in the accompanying drawings, wherein like reference numerals refer to the like elements throughout. The embodiments are described below to explain the present invention by referring to the figures.

Although the following text sets forth a detailed description of at least one embodiment or implementation, it is to be understood that the legal scope of protection of this application is defined by the words of the claims set forth at the end of this disclosure. The detailed description is to be construed as exemplary only and does not describe every possible embodiment since describing every possible embodiment would be impractical, if not impossible. Numerous alternative embodiments and/or implementations are both contemplated and possible, using either current technology or technology developed after the filing date of this patent, which would still fall within the scope of the claims.

It is to be understood that, unless a term is expressly defined in this application using the sentence "As used herein, the term" is hereby defined to mean..." or a similar sentence, there is no intent to limit the meaning of that term, either expressly or by implication, beyond its plain or ordinary meaning, and such term should not be interpreted to be limited in scope based on any statement made in any section of this patent (other than the language of the claims). To the extent that any term recited in the claims at the end of this patent is referred to in this patent in a manner consistent with a single meaning, that is done for sake of clarity only so as to not confuse the reader, and it is not intended that such claim term is limited, by implication or otherwise, to that single meaning. Finally, unless a claim element is defined by reciting the word "means" and a function without the recital of any structure, it is not intended that the scope of any claim element be interpreted based on the application of 35 U.S.C. § 112, sixth paragraph.

As used herein, "large volume of tissue" means a volume that is greater than that which may be ablated with a standard straight cryosurgical instrument, as is currently known in the art. As used herein, "standard cryosurgical instrument" means a (i.e., conventional) cryoprobe with a single cooling zone, inserted either along its main axis or approximately in that direction. One example of such a conventional cryprobe is a cryoneedle.

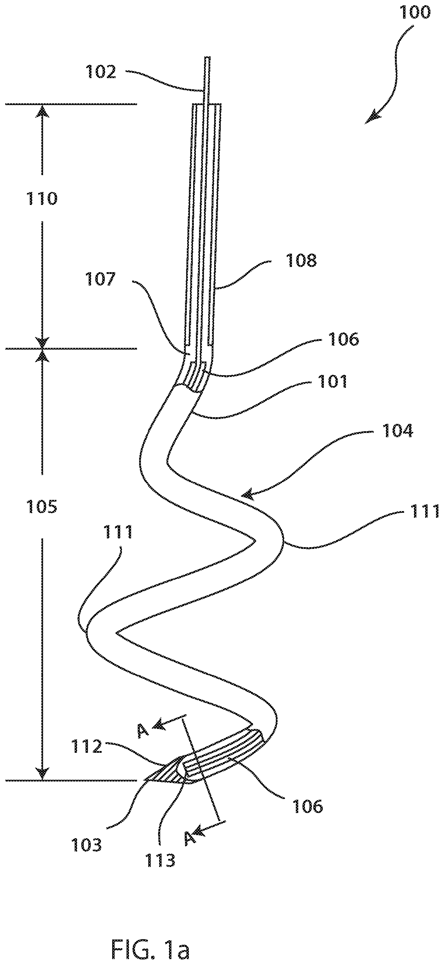

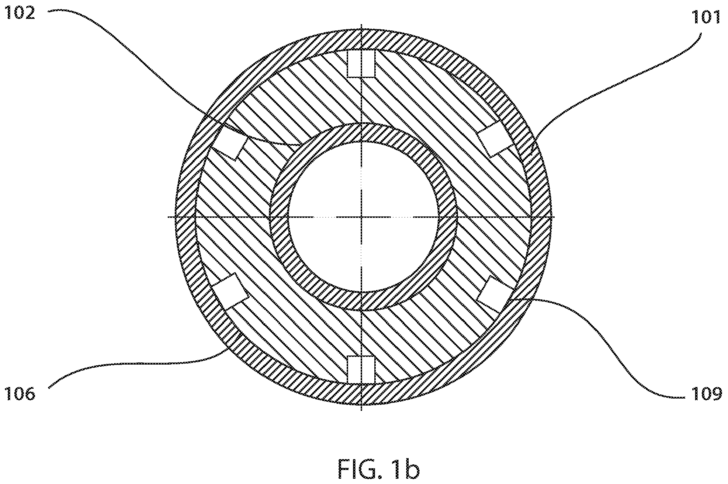

Turning now to the drawings, FIGS. 1a and 1b illustrate a cryosurgical instrument 100 that is consistent with an embodiment of the present invention. FIG. 1a shows a partial cut-away of cryosurgical instrument 100, while FIG. 1b shows a cross-section thereof taken along line A-A of FIG. 1a.

The cryosurgical instrument 100 comprises a manipulation section 110 distal to a tip 103 of the cryosurgical device and a cooling section 105 extending from the manipulating section 110 to the tip 103.

The manipulating section 110 extends along and partially defines a longitudinal axis (not shown) of the cryosurgical instrument 100. The manipulating section 110 includes insulation 108 along at least a portion of its length and a return gas sleeve 107 in gaseous communication with channels 109 to permit the exhaust of expanded/returning cryogen, as explained in detail below.

The cryosurgical instrument 100 features a helical portion 104, with surface 101 that, when inserted into a patient, is in contact with the tissue of the patient. The helical portion 104 includes one or more spirals 111, as illustrated, and terminates in a tip 103 that is preferably sharp to ease penetration into the tissue. Although two spirals are illustrated, it is to be understood that other numbers of spirals are both possible and contemplated. In this regard, the inventors have determined that a plurality of spirals 111 can be optimal in many cryosurgical applications. Further, the spirals of the helical portion 104 may be constant as illustrated or may vary in pitch and/or diameter.

The helical portion 104 may be disposed entirely within a cooling section 105 of the cryosurgical instrument 100, as shown. Alternatively, the helical portion 104 may be disposed partially beyond the cooling section 105.

Cryogen (liquid, compressed gas or mixtures of gases) enters the cryosurgical instrument 100 through one or more feeding lines 102 and travels, via a heat exchanger 106, through the cryosurgical device toward the tip 103.

Heat exchanger 106 receives the cryogen from the one or more feeding tubes 102 and delivers the cryogen to the tip 103 via port 113, which is located proximal to the tip 103. In more detail, the cryogen flows through the heat exchanger 106, thereby cooling the heat exchanger 106. And, since the heat exchanger 106 is in thermal communication with the outer surface 101, the outer surface is, in turn, cooled. In this process, the cryogen either expands or evaporates and cools, through the Joules-Thompson effect or simple evaporation. The cryogen is emitted near tip 103 through a port 113. Thereafter, the returning cryogen (cooled by the expansion, or evaporation) flows through the channels 109, which is in contact with the inner surface of the cryosurgical instrument 100, cooling the surface 101. The cryogen is then exhausted in a return sleeve 107 that preferably runs within and through the inner diameter of the insulation 108.

The inventors have discovered that for many cryosurgical applications, it can be preferable to extend the cooling section 105 to encompass the tip 103. Tip 103 may optionally comprise a reflective surface 112, as shown.

Referring specifically to FIG. 1b, the arrangement of the one or more feeding lines 102, the heat exchanger 106, the channels 109 and the cooling surface 101 are illustrated. While six channels are illustrated, it is to be understood that other numbers are both possible and contemplated.

The cooling surface 101 may optionally comprise one or more of metal, ceramic material, or plastic. The cooling section 105 is terminated at proximal end by insulation 108.

The use of the cryosurgical instrument 100 is discussed. The tip 103 of the cryosurgical instrument 100 is brought into contact with the tissue of a patient. Then sufficient force is applied to cause the tip 103 to pierce the skin of the patient. Contemporaneously or immediately thereafter, the cryosurgical instrument 100 is rotated in a manner akin to the insertion of a corkscrew into a cork so that the helical portion is embedded in the tissue. This rotational insertion causes the helical portion 104 to draw the instrument 101 toward/into the tissue to be ablated. Next, when the freezing portion 105 is positioned as desired, cryogen is supplied to cool the cooling section.

As the aforementioned description implies, the helical configuration of the cooling section 105 serves as a positioning section.

When the helical portion 104 is firmly embedded in the tissue of a patient, the spiral arrangement secures the cryosurgical instrument in the tissue. Also, when in the body, the instrument 100 can be positioned with far less forward or rearward pressure than conventional cryoprobes since the selective rotation of the helical portion 104 urges the instrument into the instrument into the body or urges the instrument out of the body. Further, the instrument 100 can be positioned with a majority of the force applied to the instrument applied to rotate the instrument.

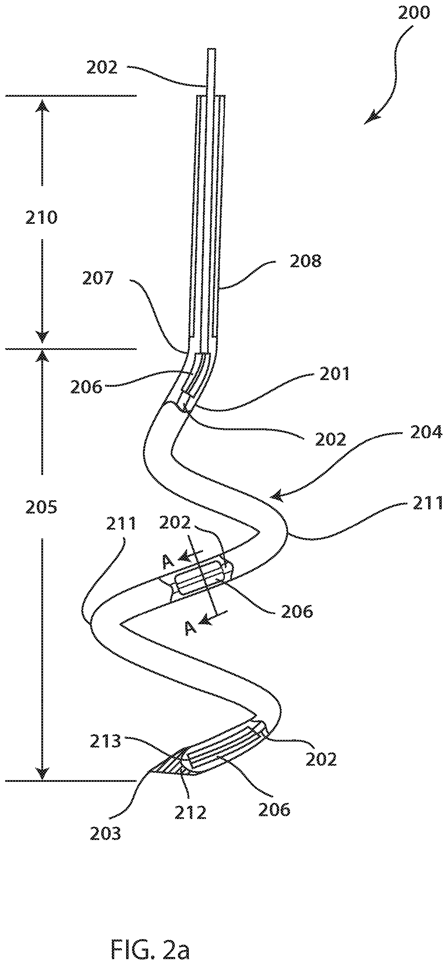

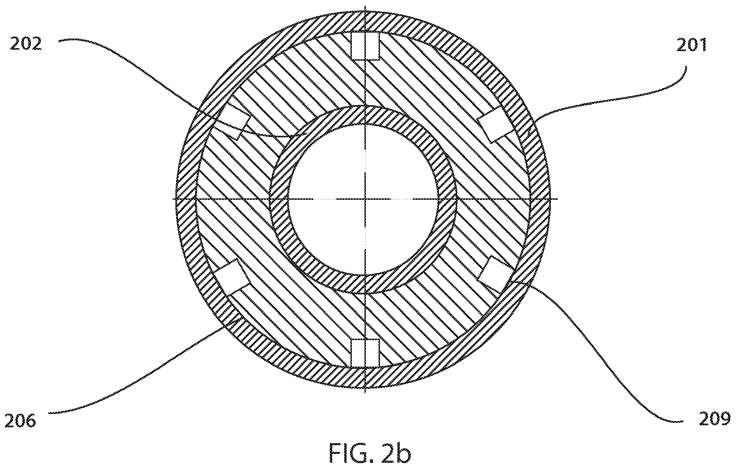

FIGS. 2a and 2B illustrate another example of a cryosurgical instrument consistent with an embodiment of the present invention. FIG. 2a shows a partial cut-away of cryosurgical instrument 200, while FIG. 2b shows a cross-section thereof taken along line A-A of FIG. 2.

In a cryosurgical instrument 200, the cryogen cools surface 201, by either expansion or evaporation, with the assistance of a plurality of discrete heat exchange elements 206, featuring grooves 209. A plurality of grooved heat exchange elements 206 is disposed in multiple locations along the cooling section 205 of the length of the cryosurgical device 200. Specific locations for the heat exchange elements depend on the desired volume of ablation.

The cryosurgical instrument 200 features a helical portion 204, with surface 201 that, when inserted into a patient, is in contact with the tissue of the patient. The helical portion 204 includes one or more spirals 211, as illustrated, and terminates in a tip 203 that is preferably sharp to ease penetration into the tissue. Although two spirals are illustrated, it is to be understood that other numbers of spirals are both possible and contemplated. In this regard, the inventors have determined that a plurality of spirals 211 can be optimal in many cryosurgical applications. Further, the spirals of the helical portion 204 may be constant as illustrated or may vary.

The helical portion 204 is disposed entirely within a cooling section 205 of the cryosurgical instrument 200, as shown. Alternatively, the helical portion 204 may be disposed partially beyond the cooling section 205.

Cryogen (liquid, compressed gas or mixtures of gases) enters the cryosurgical instrument 200 through one or more feeding lines 202 and travels to port 213, which is proximal to the tip 203.

The heat exchange elements 206 operate in a manner similar to the heat exchanger 106 of FIG. 1a, where the grooves 209 allow the flow of the cryogen into the return gas sleeve 207, thereby cooling the inner surface of cryosurgical instrument 200 at a plurality of locations as shown. Such cooling causes freezing of tissue that is in contact with the cooling surface 201. The absorption of the heat from the tissue occurs throughout the cooling section 205 by either conduction through the cooled surfaces of the grooved heat exchange elements 206 or by flow of cryogen in contact with the inner surface of cryosurgical instrument 100, up to the return fluid sleeve 207, or both. Insulation 208 keeps the manipulation section 210 from being cooled by the cryogen.

The inventors have discovered that for many cryosurgical applications, it can be preferable to extend the cooling section 205 to encompass the tip 203. Tip 203 may optionally comprise a reflective surface 212, as shown.

Preferably the distribution of the grooved heat exchange elements is sufficient to yield a single, continuous ablations zone. However, the inventors also contemplate distributions that provide discrete ablations zones along segments of the length of the helical portion 204.

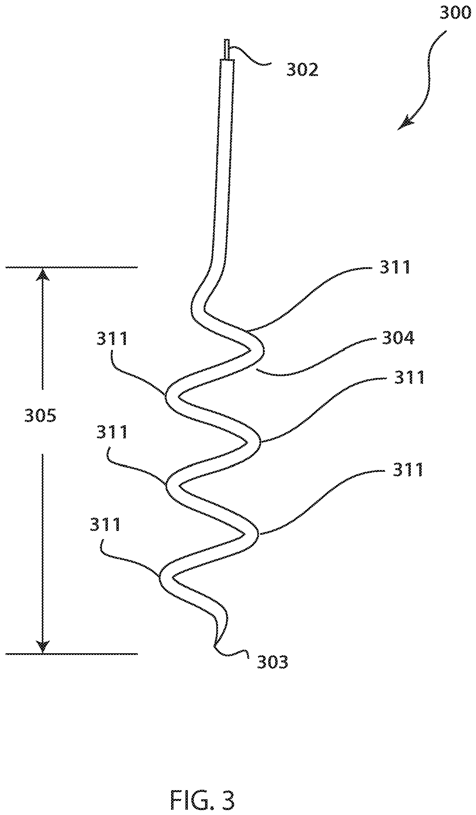

Figure 3 shows another example of another cryosurgical instrument consistent with an embodiment of the present invention.

The cryosurgical instrument 300 features a helical portion 304 that includes several spirals 311 such that the freezing portion spirals more than 360 degrees. Optionally any type of heat exchange element, such as the above-described grooves may be employed (not shown). The surface area in the cooling section 305 is therefore greater than the equivalent cooling surface of the systems of FIGS. 1a-2b.

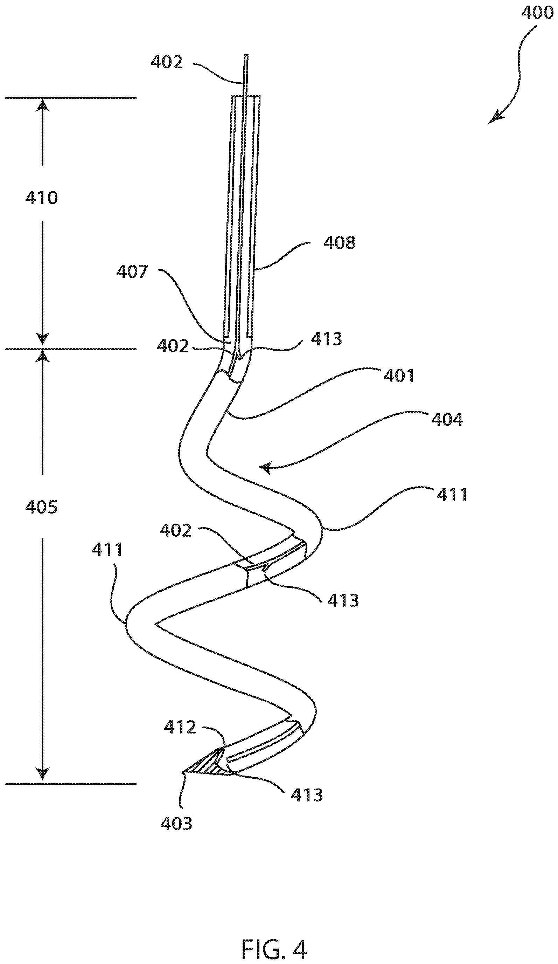

FIG. 4 shows another example of a cryosurgical instrument consistent with an embodiment of the present invention. In cryosurgical instrument 400, the cryogen may optionally comprise any type of fluid and can be adapted for Joule-Thomson cooling. As is known in the art, Joule-Thomson cooling is based upon the Joule-Thomson effect, in which a compressed fluid is forced through a narrow opening, resulting in rapid expansion of the compressed fluid, and cooling of the fluid.

The cryosurgical instrument 400 features a helical portion 404, with surface 401 that, when inserted into a patient, is in contact with the tissue of the patient. The helical portion 404 includes one or more spirals 411, as illustrated, and terminates in a tip 403 that is preferably sharp to ease penetration into the tissue. Although two spirals are illustrated, it is to be understood that other numbers of spirals are both possible and contemplated. In this regard, the inventors have determined that a plurality of spirals 411 can be optimal in many cryosurgical applications. Further, the spirals of the helical portion 404 may be constant as illustrated or may vary.

The helical portion 404 is disposed entirely within a cooling section 405 of the cryosurgical instrument 400.

Cryogen (liquid, compressed gas or mixtures of gases) enters the cryosurgical instrument 400 through cryogen feed line 402 and travels toward the tip 103.

To support the Joule-Thomson effect, cryosurgical instrument 400 preferably features one or more adiabatic nozzles 413 to introduce the fluid cryogen into the inner area of cryosurgical instrument 400. Upon expansion or evaporation of the cryogen, depending on the type of fluid, the surface 401 is cooled generating the cooling section 405. Optionally, nozzle(s) 413 may be provided as ports or other openings (not shown).

Alternatively, if one adiabatic expansion nozzle 413 is used, the adiabatic expansion nozzle 413 is preferably located at or near tip 403. If a plurality of nozzles 413 is used, such adiabatic expansion nozzles 406 are preferably installed at multiple locations along the inlet feeding tube 402, more preferably distributed more or less equally. Such one or more adiabatic expansion nozzles 413 let part, or all, of the pressurized cryogen flowing in the cryogen feed line 402, to exit and expand. The cryogen feed line 402 delivers the cryogen, or portion of it if a plurality of nozzles 413 is present, to the tip 403.

The inventors have discovered that for many cryosurgical applications, it can be preferable to extend the cooling section 405 to encompass the tip 403. Tip 403 may optionally comprise a reflective surface 412, as shown. In addition, heat regenerating coils, not shown, may be added within the supply tube 402 and/or at each port 406, to cool the compressed fluid before it is ejected from each port.

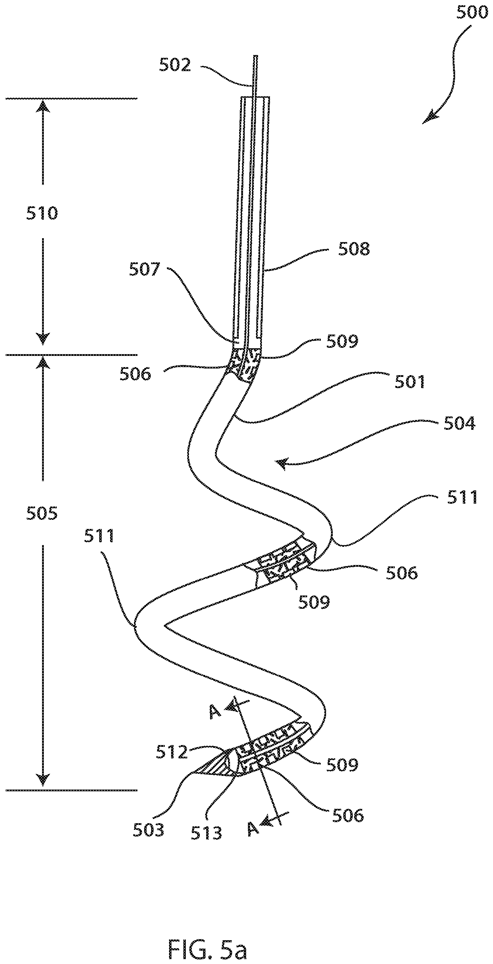

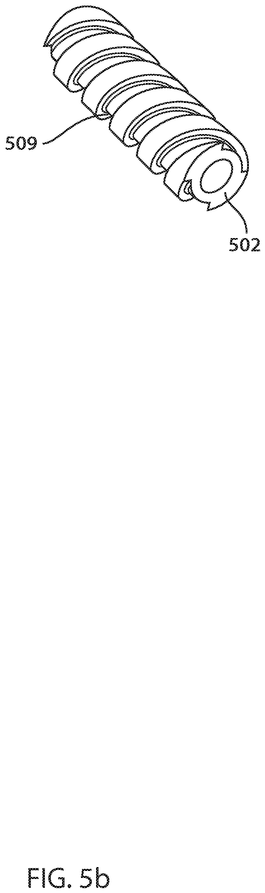

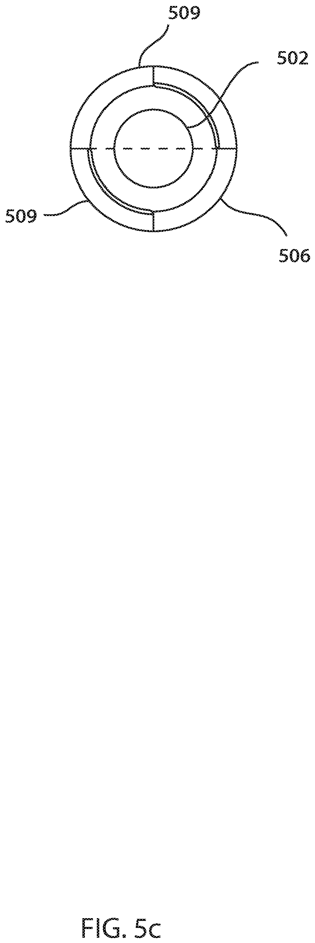

FIGS. 5a-5c show an example of another cryosurgical instrument consistent with an embodiment of the present invention. FIG. 5a shows a partial cut-away view of the cryosurgical instrument 500. FIG. 5b is a perspective view of the helical grooves 509 in relation to cryogen supply line 502. FIG. 5c is a cross-sectional view taken along line A-A of FIG. 5a.

The cryosurgical instrument 500 features a helical portion 504, with surface 501 that, when inserted into a patient, is in contact with the tissue of the patient. The helical portion 504 includes one or more spirals 511, as illustrated, and terminates in a tip 503 that is preferably sharp to ease penetration into the tissue. Although two spirals are illustrated, it is to be understood that other numbers of spirals are both possible and contemplated. In this regard, the inventors have determined that a plurality of spirals 511 can be optimal in many cryosurgical applications. Further, the spirals of the helical portion 504 may be constant as illustrated or may vary.

The helical portion 504 is disposed entirely within a cooling section 505 of the cryosurgical instrument 500.

Cryogen (liquid, compressed gas or mixtures of gases) enters the cryosurgical instrument 500 through cryogen feed line 502 and travels to the tip 503.

In cryosurgical instrument 500, a plurality of discrete heat exchange elements 506 are disposed at various locations along a cryogen supply line 502 in a cooling zone 505. Preferably each of the heat exchange elements 506 comprises a plurality of helical channels 509. The cryogen supplied by input feeding line 502 then flows back to the return sleeve 507, cooling the surface 501 by either direct contact of the cryogen flowing along the spaces created between the helical channels 509 and the outer surface of the instrument, or by conduction of heat via the contact surfaces of heat exchange elements 506 and the inner surface of cryosurgical instrument 500, thereby cooling surface 501.

As shown in Fig. 5b, the channels 509 spiral about the cryogen supply line.

Although only a single cryogen supply line 502 is shown, it is to be understood that multiple cryogen supply lines are both possible and contemplated. Also, when multiple cryogen supply lines 502 are present, each line may have multiple heat exchange elements 506.

The inventors have discovered that for many cryosurgical applications, it can be preferable to extend the cooling section 505 to encompass the tip 503. Tip 503 may optionally comprise a reflective surface 512, as shown.

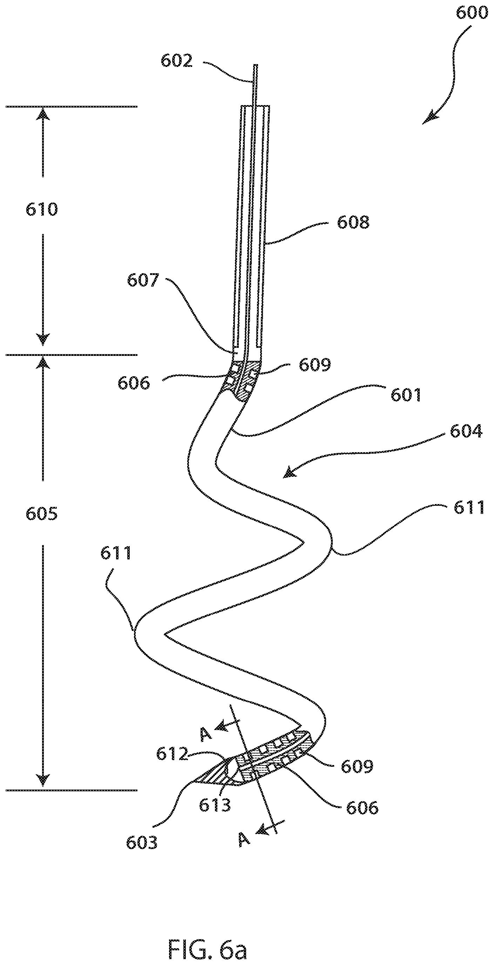

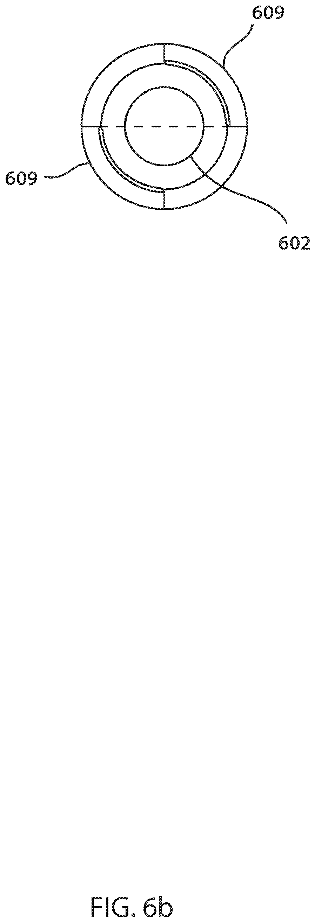

FIGS. 6a and 6b show another example of cryosurgical instrument consistent with an embodiment of the present invention. FIG. 6a shows a partial cut-away view while FIG. 6b shows a cross-sectional view along line A-A of FIG. 6a.

The cryosurgical instrument 600 features a helical portion 604, with surface 601 that, when inserted into a patient, is in contact with the tissue of the patient. The helical portion 604 includes one or more spirals 611, as illustrated, and terminates in a tip 603 that is preferably sharp to ease penetration into the tissue. Although two spirals are illustrated, it is to be understood that other numbers of spirals are both possible and contemplated. In this regard, the inventors have determined that a plurality of spirals 611 can be optimal in many cryosurgical applications. Further, the spirals of the helical portion 604 may be constant as illustrated or may vary.

The helical portion 604 is disposed entirely within a cooling section 605 of the cryosurgical instrument 600.

The cryosurgical instrument 600 includes a cryogen feeding input line 602 to feed cryogen to a single heat exchange element 606 having helical channels 609 that extend from a tip 603 to a return gas sleeve 607. In operation, cryogen enters the cryosurgical instrument 600 via input line 602, cools the heat exchanger 606, is discharged through a port 613 proximal to the tip 603, flows back through the helical channels 609 to the return fluid sleeve 607 thereby cooling the surface 601 by either: (1) direct contact of the cryogen flowing along the helical groove 609; or (2) conduction of heat via the contact surface of heat exchange element 606 and the inner surface of cryosurgical instrument 600 thereby cooling the surface 601, throughout the cooling zone 605.

Referring to FIG. 6b, the relationship between the spiral grooves 609 and the feed line 602 are illustrated.

The inventors have discovered that for many cryosurgical applications, it can be preferable to extend the cooling section 605 to encompass the tip 603. Tip 603 may optionally comprise a reflective surface 612, as shown.

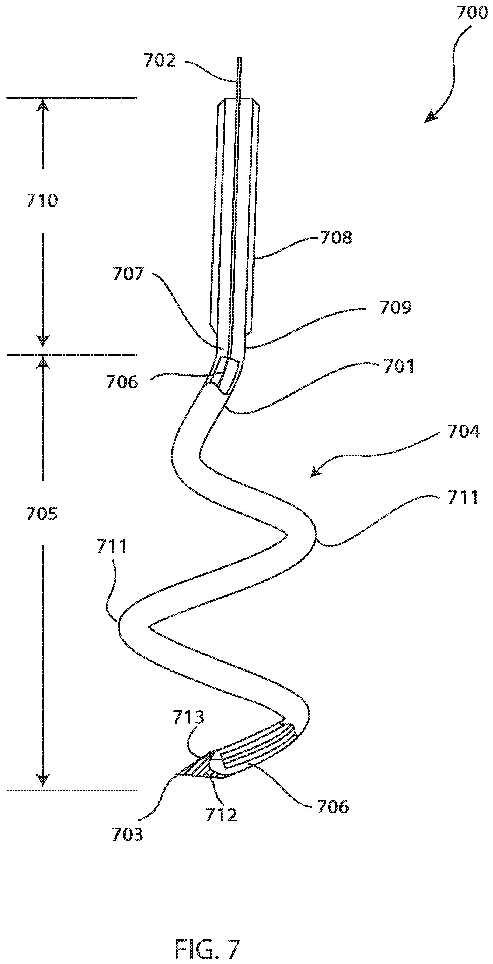

FIG. 7 shows another example of a cryosurgical instrument consistent with an embodiment of the present invention. The cryosurgical instrument 700 features insulation 708 disposed on the outer surface of the cryosurgical device in the manipulation zone 710. In this example, a cryogen feeding input line 702 feeds cryogen to a single heat exchange element 706, which delivers the cryogen to port 713 located proximal to the tip 703.

In more detail, the cryosurgical instrument 700 features a helical portion 704, with surface 701 that, when inserted into a patient, is in contact with the tissue of the patient. The helical portion 704 includes one or more spirals 711, as illustrated, and terminates in a tip 103 that is preferably sharp to ease penetration into the tissue. Although two spirals are illustrated, it is to be understood that other numbers of spirals are both possible and contemplated. In this regard, the inventors have determined that a plurality of spirals 711 can be optimal in many cryosurgical applications. Further, the spirals of the helical portion 704 may be constant as illustrated or may vary.

The helical portion 704 is disposed entirely within a cooling section 705 of the cryosurgical instrument 700.

The inventors have discovered that for many cryosurgical applications, it can be preferable to extend the cooling section 705 to encompass the tip 703. Tip 703 may optionally comprise a reflective surface 712, as shown.

The inventors have discovered that for many cryosurgical applications, it can be preferable to extend the cooling section 705 to encompass the tip 703. Tip 703 may optionally comprise a reflective surface 712, as shown.

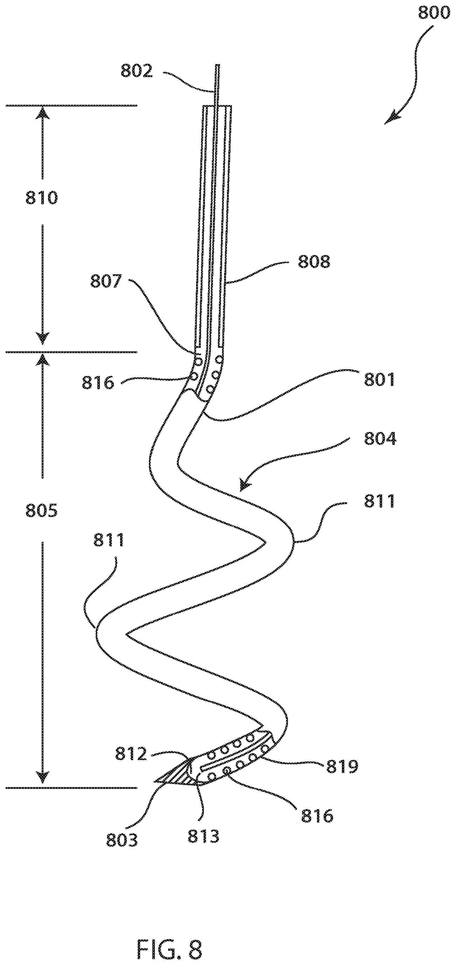

FIG. 8 shows an example of another cryosurgical instrument 800 consistent with an embodiment of the present invention.

The cryosurgical instrument 800 features a helical portion 804, with surface 801 that, when inserted into a patient, is in contact with the tissue of the patient. The helical portion 804 includes one or more spirals 811, as illustrated, and terminates in a tip 803 that is preferably sharp to ease penetration into the tissue. Although two spirals are illustrated, it is to be understood that other numbers of spirals are both possible and contemplated. In this regard, the inventors have determined that a plurality of spirals 811 can be optimal in many cryosurgical applications. Further, the spirals of the helical portion 804 may be constant as illustrated or may vary.

The helical portion 804 is disposed entirely within a cooling section 805 of the cryosurgical instrument 800.

In cryosurgical instrument 800, a cryogen feeding input line 802 delivers cryogen to a tip 803. The cryosurgical instrument 800 also includes a barrier 816 that urges the flow of returning cryogen toward outer surface 801 of the cryosurgical instrument 800so as to cool surface 801. Barrier 816 effectively forces the return fluid to flow in an open space 819 between the barrier 816 from tip 803 to the return gas sleeve 807, creating the cooling zone 805. The cooling zone 805 ends at insulation 808 that is located in manipulation zone 810.

The barrier 816 is illustrated as a coiled tube. However, it is to be understood that other configurations are both possible and contemplated. Indeed, the barrier 816 need not have the round cross-section as shown. Rather, any cross-section that urges the return flow towards the outer surface may be used. Also, the barrier may spiral with constant pitch as illustrated. Alternatively, the barrier 816 may spiral at a varied pitch.

The inventors have discovered that for many cryosurgical applications, it can be preferable to extend the cooling section 805 to encompass the tip 803. Tip 803 may optionally comprise a reflective surface 812, as shown.

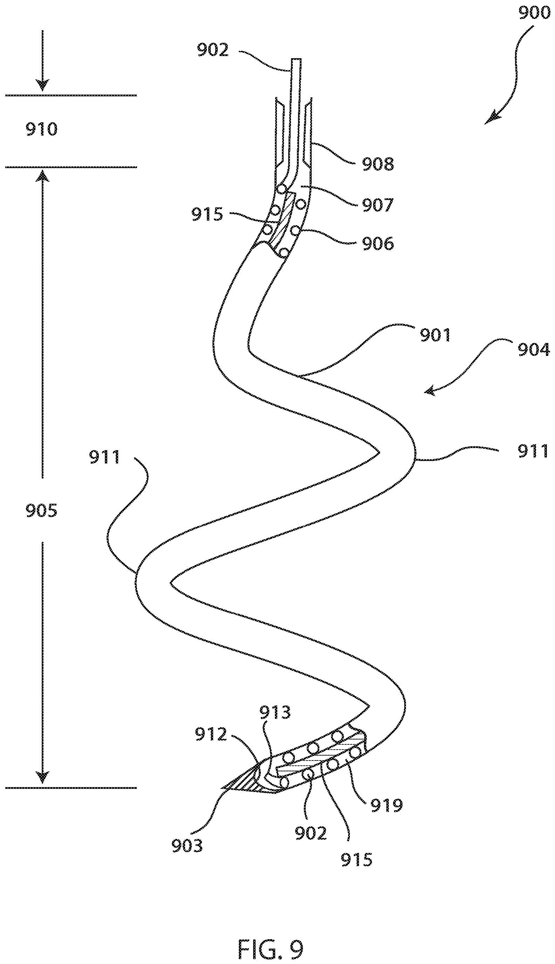

FIG. 9 shows an example of a cryosurgical instrument 900 consistent with an embodiment of the present invention.

The cryosurgical instrument 900 features a helical portion 904, with surface 901 that, when inserted into a patient, is in contact with the tissue of the patient. The helical portion 904 includes one or more spirals 911, as illustrated, and terminates in a tip 903 that is preferably sharp to ease penetration into the tissue. Although two spirals are illustrated, it is to be understood that other numbers of spirals are both possible and contemplated. In this regard, the inventors have determined that a plurality of spirals 911 can be optimal in many cryosurgical applications. Further, the spirals of the helical portion 904 may be constant as illustrated or may vary.

The helical portion 904 is disposed entirely within a cooling section 905 of the cryosurgical instrument 800.

In cryosurgical instrument 900, a cryogen feeding input line 902 is coiled around a solid core 915, from the return fluid sleeve 907 to the tip 903, ending with port 913 at which the cryogen is left to flow within the cooling surface 901. In operation, cryogen flowing in the coiled line 902 cools the surface of the coiled tube 902. This surface, in turn, cools the inner surface of the cryosurgical instrument 900, which, in turn, is in thermal communication with and cools the cooling surface 901. After the cryogen exits the supply line 902 at a port 913, it flows back through the gap 919 created by the inlet tube 902 as a barrier next to the inner surface of cryosurgical instrument 900.

The cryogen supply line 902 may spiral at a constant rate as illustrated. Alternatively, the cryogen supply line 902 may spiral at a varied rate.

The inventors have discovered that for many cryosurgical applications, it can be preferable to extend the cooling section 905 to encompass the tip 903. The cooling zone 905 ends at insulation 908 that is located in a manipulation section (not shown). Tip 903 may optionally comprise a reflective surface 912, as shown.

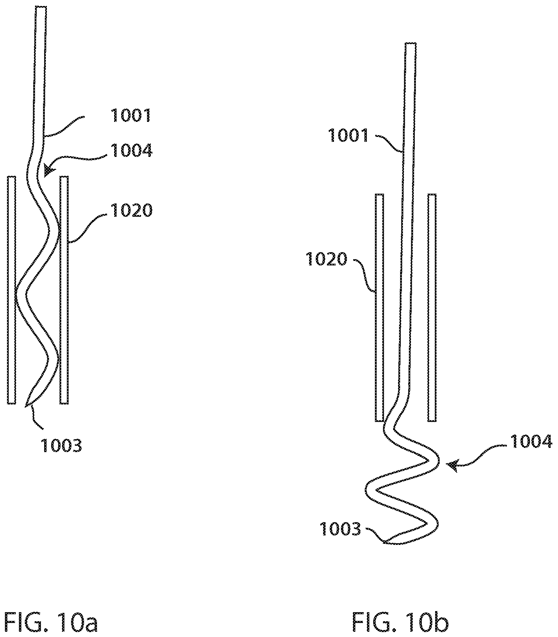

FIGS. 10a and 10b show an example of a system that includes a cryosurgical instrument consistent with an embodiment of the present invention. FIG. 10a illustrates a system 1000 with a flexible cryosurgical instrument 1001. The instrument 1001 includes a helical section 1004 and a sharp tip 1003. The instrument 1001 is selectively retractable into (i.e., drawn into) a sleeve 1020 of, for example a trocar, and selectively protractable from (i.e., extendable from) the sleeve 1020. To facilitate this functionality, the instrument 1001 is flexible and compressible. Optionally, the cryosurgical instrument may be made of material with memory.

Following the insertion of the cryosurgical instrument 1001 through the sleeve 1020, the mechanical flexibility of the instrument 1001, an applied pressure, and/or a temperature increase, causes the instrument to expand from the retracted condition shown in FIG. 10a to the protracted condition as shown in FIG. 10b. Cryosurgical instrument 1001 may be any instrument consistent with any embodiment of the present invention.

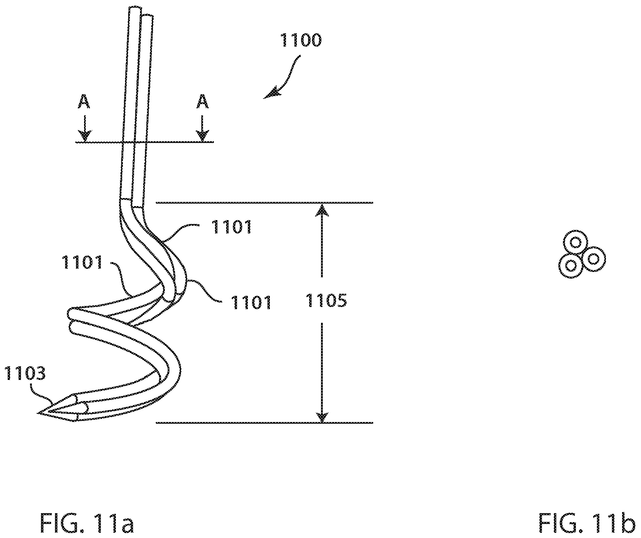

Figure 11 shows an example of another cryosurgical instrument consistent with an embodiment of the present invention. FIG. 11a shows a side view of cryosurgical instrument 1100, while FIG. 1b shows a cross-section thereof taken along line A-A of FIG. 11a.

Cryosurgical instrument 1100 features a plurality of individual cryosurgical instruments 1101 arranged in a cluster, each with the same pitch and outside diameter. The plurality may include three instruments, as illustrated, although other numbers are both contemplated and possible. Also, all of the instruments may be the same instrument as illustrated, or a combination of different cryosurgical instruments.

Each cryosurgical instrument 1101 may be any instrument consistent with any embodiment of the present invention.

The cooling sections 1105 of each instrument 1101 may spiral about the longitudinal axis at a diameter and pitch that differs from those of the other cooling sections of the other instruments. Alternatively, the cooling sections 1105 of each instrument 1101 may spiral about the longitudinal axis at a diameter and pitch that is the same as those of the other cooling sections of the other instruments.

The use of each of cryosurgical instruments 200, 300, 400, 500, 600, 700, 800, 900, 1000, and 1101 is substantially similar to that of cryosurgical instrument 100.

As the foregoing shows, a specially shaped cryosurgical instrument has been devised that facilitates: placing several cryoprobes, or cryo-coolers, closely in relation to one another; positioning them in flexible unfixed organ; and eliminating the need for a guide. The novel cryosurgical instrument penetrates the tissue by rotation. The treatment of the large volume of tissue is achieved by either several cooling elements, prepositioned, or by cooling the whole surface of defined ablation zone. The prepositioned ablating elements eliminate the needed skill of the physician to place several small cryosurgical instruments in relation to one another to create continuous volume of ablation. Consequently, the need to penetrate the tissue in several places is avoided. In addition, because of its shape, the novel instrument is anchored in the tissue to retain it in its position during thawing part of the process without an additional holding force.

An aspect of the present invention provides a cryosurgical instrument including a helical element that receives a cryogenic fluid and a tip that is cooled by the cryogenic fluid. The portion of the instrument that is cooled by the cryogenic fluid may optionally include an extended distal section of the instrument that, together with the tip, is a cooling zone. Upon insertion to a tissue the cooling zone is cooled and causes an ice ball to form, thereby causing cryoablation of the tissue volume defined by the ablation zone of the instrument.

As the foregoing also shows, cryosurgical instruments according to at least some embodiments of the present invention overcome the above drawbacks of the background art, including but not limited to, the requirement to insert several cryosurgical instrument in a defined spatial volume and in relation to each other, to position such cryosurgical instruments in a flexible unfixed organ, and the need for a template or guiding element.

Cryosurgical instruments according to at least some embodiments of the present invention, featuring at least one helical element, penetrate the tissue by rotation. By turning the instrument, the tip and the body of the instrument penetrate deep into the tissue. The treatment of the large volume of tissue is achieved by either several cooling elements, prepositioned within or attached to the cryosurgical instrument, or by cooling the whole surface of defined ablation zone. The prepositioned ablating elements eliminate the needed skill of the physician to place several small cryosurgical instruments in relation to one another to create a continuous volume of ablation. Another advantage is the creation of a single hole, rather than multiple holes as for the background art instruments. This instrument is also, inherently from its shape, anchored in the tissue and optionally no additional holding force is required to retain it in its position during thawing part of the process.

As previously described, at least a portion of the cryosurgical instrument is in the shape of a spiral or corkscrew element, with a tip that is preferably a sharp penetrating tip. As the instrument enters the tissue to be ablated, rotation of the instrument causes the penetration thereof into the tissue. The depth of the penetration is determined by the size of the corkscrew element; however the freezing ablation zone volume is extended further out and forwards depending on the pitch and outside diameter of the spiral and the cooling capacity. The freezing element may either utilize the Joule-Thomson effect caused by expanded high-pressure gas, or evaporating liquefied gas. Heating the element to either thaw the frozen tissue or to release the instrument from the tissue, can be done by either supplying high pressure gas that heats upon expansion (Joule-Thomson method), supplying a heated gaseous form of the liquefied freezing cryogen, or supplying electrical power to specially placed heating elements.

The use of various ones of the above-described examples is discussed. Generally, the shape of the cryosurgical instrument is a spiral type, with a sharp penetrating tip. As the instrument brought into contact with the desired ablated volume, the instrument is rotated, which causes the penetration of the tissue since the turning causes a forward motion like "cork screw". The size of the penetration is the size of external tube; however the freezing ablation zone is extended further outward and forward depending on the pitch and outside diameter of the spiral and the cooling capacity. The freezing element may either utilize the Joule-Thomson effect caused by expanded high-pressure gas, or evaporating liquefied gas. Heating the element to either thaw the frozen tissue or to release the instrument from the tissue, can be done by either supplying high pressure gas which heats upon expansion (Joule-Thomson effect), supplying a heated gaseous form of the liquefied freezing cryogen, or supplying electrical power to specially placed heating elements.

Examples of various features/aspects/components/operations have been provided to facilitate understanding of the disclosed embodiments of the present invention. In addition, various preferences have been discussed to facilitate understanding of the disclosed embodiments of the present invention. It is to be understood that all examples and preferences disclosed herein are intended to be nonlimiting.

Although selected embodiments of the present invention have been shown and described individually, it is to be understood that at least aspects of the described embodiments may be combined.

Although selected embodiments of the present invention have been shown and described, it is to be understood the present invention is not limited to the described embodiments. Instead, it is to be appreciated that changes may be made to these embodiments without departing from the principles of the invention, the scope of which is defined by the claims and the equivalents thereof.

## Claims

1. A cryosurgical instrument (100) that is selectively configured for being positioned in a patient by rotation, comprising:
a manipulation section (110) that permits a user to rotate the instrument; and
a cryogen supply portion,
a positioning section having a sharp tip at an end thereof; the cryosurgical instrument **characterized by**:
a plurality of cryogen supply lines (102) that supply cryogen to the instrument (100); two or more discrete heat exchangers (106) disposed along each of the one or more cryogen supply lines (102) and separated by a length of the one or more supply lines, wherein each heat exchanger comprises channels that spiral about each supply line;
a helical portion (104), the helical portion (104) of the cryosurgical instrument (100) having an outer surface (101) in thermal communication with the heat exchangers (106), wherein the heat exchangers (106) are located within the helical portion (104) of the cryosurgical instrument (100), the heat exchangers (106) are configured to receive cryogen from the cryogen supply portion and to permit the received cryogen to cool the helical portion (104) of the cryosurgical instrument (100).

2. The cryosurgical instrument of claim 1, wherein the helical portion (104) is configured to
urge the cryosurgical instrument (100) into the patient when the instrument (100) is rotated in a first direction, via the manipulation section (110), and
urge the cryosurgical instrument (100) out of the patient when the instrument (100) is rotated in a second direction that is opposite the first, via the manipulation section (110),
wherein the cryosurgical instrument (100) is configured to travel in a direction along a longitudinal axis thereof when the instrument is rotated, via the manipulation section (110), without a majority of a positioning force being applied to the cryosurgical instrument (100) in the direction.

3. The cryosurgical instrument (100) of claim 1 wherein:
the outer surface (101) is a substantially smooth, temperature conducting outer surface (101) with an inner side, the cryosurgical instrument (100) , wherein:
at least a portion of the manipulation section (110) extends along and defines a longitudinal axis of the instrument (100), the manipulating section having insulation (108) along at least a portion of a length thereof, and
a return fluid sleeve (107) to permit exhaust of expanded fluid from the instrument (100);
and each heat exchanger (106) is in fluid communication with the one or more cryogen supply lines (102), surrounds the one or more cryogen supply lines (102) in the helical portion (104), and delivers received cryogen to a port at a distal end thereof proximal to the tip (103), wherein each channel (109) is circumferentially disposed along the inner side of the outer surface (101) and that interconnect the one or more supply lines to the return gas sleeve (107), each heat exchanger (106) permitting cryogen from the one or more supply lines to cool a cooling section; and the cryosurgical instrument (100) further comprises:
an expanded fluid return pathway surrounding the one or more cryogen supply lines (102) and bounded by the one or more cryogen supply lines (102) and the inner side of the outer surface (101) of the instrument (100) so that gas in the pathway is in thermal communication with the inner side.

4. The cryosurgical instrument (100) of claim 3, wherein the helical portion (104) includes multiple spirals that spiral around the longitudinal axis more than 360 degrees.

5. The cryosurgical instrument (100) of claim 3, wherein the helical portion (104) is flexible and has a shape memory.

6. The cryosurgical instrument (100) of claim 6, wherein the helical portion (104) is formed with Nitinol, spring steel, or a flexible steel.

7. The cryosurgical instrument (100) of claim 1, wherein a rate of the spiraling of the cryogen supply line (102) is constant.

8. The cryosurgical instrument (100) of claim 3, wherein:
the helical portion spirals around the longitudinal axis at a pitch that varies; or
a diameter of the spiraling of the helical portion varies.

9. A system (1100) comprising multiple ones of the cryosurgical instruments (1101; 100) of claim 3, wherein the instruments (1101; 100) are clustered together along their lengths.

10. The cryosurgical instrument (100) of claim 3, wherein:
each supply line of the one or more cryogen supply lines (102) delivers cryogen to one or more ports along a portion of a length thereof in the helical portion, at least one of the supply lines has a port at a distal end thereof proximal to the tip (103);
and the return fluid sleeve (107) surrounds the one or more cryogen supply lines (102) and is bounded by the one or more cryogen supply lines and the inner side of the outer surface (101) of the instrument (100) so that fluid in the sleeve is in thermal communication with the inner side, the sleeve permitting cryogen from the one or more supply lines (102) to cool the helical portion.

11. The cryosurgical instrument (100) of claim 1, wherein the outer surface (101) is substantially smooth, temperature conducting, and has an inner side, the cryosurgical instrument (100) comprising:
each cryogen supply line (102) delivers cryogen to a port at an end proximal to the tip (103), wherein:
at least a portion of the manipulation section (110) extending along and defining a longitudinal axis of the instrument, and the manipulating section has
insulation (108) along at least a portion of a length thereof, and
a return fluid sleeve (107) to permit exhaust of expanded gas from the instrument (100);
a cooling section connected to the manipulation section (110), extending from the manipulation section (110) to the tip (103), and comprising
the helical portion, wherein the helical portion has a helical configuration spiraling around the longitudinal axis, and
a barrier that is disposed only in the cooling section and spirals about the cryogen supply line (102), the barrier yielding a spiraling channel that is circumferentially disposed along the inner side of the outer surface (101), that is bounded by the cryogen supply line (102) and the inner side of the outer surface (101) of the instrument, and that interconnects the supply line to the return gas sleeve (107), the barrier permitting cryogen exiting the port to cool the cooling section.

12. The cryosurgical instrument (100) of claim 3, comprising:
a core that is disposed only in the helical portion and that extends from the manipulating section to substantially near the tip (103), wherein
the cryogen supply line (102) spirals around the core, and wherein
the spiraling of the cryogen supply line (102) yields a spiraling channel that is circumferentially disposed along the inner side of the outer surface (101), that is bounded by the cryogen supply line (102) and the inner side of the outer surface (101) of the instrument (100), and that interconnects the supply line (102) to the a return fluid sleeve, the spiraling channel permitting cryogen exiting the port to cool the helical portion.

## Patentansprüche

1. Kryochirurgisches Instrument (100), das selektiv konfiguriert ist, um in einem Patienten durch Drehen positioniert zu werden, umfassend:
einen Handhabungsabschnitt (110), der es einem Benutzer ermöglicht, das Instrument zu drehen; und
einen Kryogenzufuhrteil,
einen Positionierungsabschnitt mit einer scharfen Spitze an einem Ende davon; wobei das kryochirurgische Instrument **gekennzeichnet ist durch**:
mehrere Kryogenzuleitungen (102), die dem Instrument (100) Kryogen zuführen; zwei oder mehr separate Wärmetauscher (106), die an jeder der einen oder mehr Kyrogenzuleitungen (102) entlang angeordnet sind und **durch** ein Stück der einen oder mehr Zuleitungen getrennt sind, wobei jeder Wärmetauscher Kanäle aufweist, die sich um jede Zuleitung winden;
einen Wendelteil (104), wobei der Wendelteil (104) des kryochirurgischen Instruments (100) eine Außenfläche (101) hat, die mit den Wärmetauschern (106) in thermischer Verbindung steht, wobei die Wärmetauscher (106) sich innerhalb des Wendelteils (104) des kryochirurgischen Instruments (100) befinden, die Wärmetauscher (106) dabei konfiguriert sind, um Kryogen aus dem Kryogenzufuhrteil zu erhalten und das erhaltene Kryogen den Wendelteil (104) des kryochirurgischen Instruments (100) kühlen zu lassen.

2. Kryochirurgisches Instrument nach Anspruch 1, wobei der Wendelteil (104) konfiguriert ist, um
das kryochirurgische Instrument (100) in den Patienten zu drängen, wenn das Instrument (100) über den Handhabungsabschnitt (110) in einer ersten Richtung gedreht wird, und
das kryochirurgische Instrument (100) aus dem Patienten heraus zu drängen, wenn das Instrument (100) über den Handhabungsabschnitt (110) in einer zweiten Richtung gedreht wird, die der ersten entgegengesetzt ist,
wobei das kryochirurgische Instrument (100) konfiguriert ist, um sich in einer Richtung entlang einer Längsachse davon zu bewegen, wenn das Instrument über den Handhabungsabschnitt (110) gedreht wird, ohne dass eine Mehrheit einer Positionierungskraft in der Richtung auf das kryochirurgische Instrument (100) angewendet wird.

3. Kryochirurgisches Instrument (100) nach Anspruch 1, wobei:
die Außenfläche (101) eine im Wesentlichen glatte, thermisch leitende Außenfläche (101) mit einer Innenseite ist, wobei bei dem kryochirurgischen Instrument (100):
wenigstens ein Teil des Handhabungsabschnitts (110) an einer Längsachse des Instruments (100) entlang verläuft und dieselbe definiert, wobei der Handhabungsteil Folgendes hat:
Isolierung (108) an wenigstens einem Teil seiner Länge entlang und
eine Rückfluidhülse (107), um expandiertes Fluid aus dem Instrument (100) abzulassen;
und jeder Wärmetauscher (106) mit der einen oder den mehr Kryogenzuleitung(en) (102) in Fluidverbindung ist, die eine oder mehr Kryogenzuleitung(en) (102) im Wendelteil (104) umgibt und erhaltenes Kryogen zu einer Öffnung an einem distalen Ende davon, proximal der Spitze (103), liefert, wobei jeder Kanal (109) entlang der Innenseite der Außenfläche (101) am Umfang angeordnet ist, und sie die eine oder mehr Zuleitung(en) mit der Rückgashülse (107) verbinden, wobei jeder Wärmetauscher (106) Kryogen aus der einen oder den mehr Zuleitung(en) einen Kühlabschnitt kühlen lässt; und das kryochirurgische Instrument (100) ferner Folgendes aufweist:
eine Rückströmbahn für expandiertes Fluid, die die eine oder mehr Kryogenzuleitung(en) (102) umgibt und von der einen oder den mehr Kryogenzuleitung(en) (102) und der Innenseite der Außenfläche (101) des Instruments (100) umgrenzt ist, sodass Gas in der Bahn mit der Innenseite in thermischer Verbindung steht.

4. Kryochirurgisches Instrument (100) nach Anspruch 3, wobei der Wendelteil (104) mehrere Windungen beinhaltet, die mehr als 360 Grad um die Längsachse gewunden sind.

5. Kryochirurgisches Instrument (100) nach Anspruch 3, wobei der Wendelteil (104) flexibel ist und ein Formgedächtnis hat.

6. Kryochirurgisches Instrument (100) nach Anspruch 6, wobei der Wendelteil (104) aus Nitinol, Federstahl oder einem flexiblen Stahl hergestellt ist.

7. Kryochirurgisches Instrument (100) nach Anspruch 1, wobei eine Windungsrate der Kryogenzuleitung (102) konstant ist.

8. Kryochirurgisches Instrument (100) nach Anspruch 3, wobei:
der Wendelteil sich mit einer Ganghöhe, die variiert, um die Längsachse windet oder ein Durchmesser der Windungen des Wendelteils variiert.

9. System (1100), das mehrere der kryochirurgischen Instrumente (1101; 100) nach Anspruch 3 aufweist, wobei die Instrumente (1101; 100) längs an ihnen entlang zusammengeschart sind.

10. Kryochirurgisches Instrument (100) nach Anspruch 3, wobei:
jede Zuleitung der einen oder mehr Kryogenzuleitung(en) (102) Kryogen zu einer oder mehr Öffnung(en) entlang einem Teil eines Stücks davon im Wendelteil liefert, wobei wenigstens eine der Zuleitungen an einem distalen Ende davon proximal der Spitze (103) eine Öffnung hat;
und die Rückfluidhülse (107) die eine oder mehr Kryogenzuleitung(en) (102) umgibt und von der einen oder den mehr Kryogenzuleitung(en) und der Innenseite der Außenfläche (101) des Instruments (100) umgrenzt ist, sodass Fluid in der Hülse mit der Innenseite in thermischer Verbindung steht, wobei die Hülse Kryogen aus der einen oder den mehr Zuleitung(en) (102) den Wendelabschnitt kühlen lässt.

11. Kryochirurgisches Instrument (100) nach Anspruch 1, wobei die Außenfläche (101) im Wesentlichen glatt ist, thermisch leitend ist und eine Innenseite hat, wobei das kryochirurgische Instrument (100) Folgendes aufweist:
jede Kryogenzuleitung (102) liefert Kryogen zu einer Öffnung an einem zur Spitze (103) proximalen Ende, wobei:
wenigstens ein Teil des Handhabungsabschnitts (110) an einer Längsachse des Instruments entlang verläuft und dieselbe definiert und der Handhabungsteil Folgendes hat:
Isolierung (108) an wenigstens einem Teil seiner Länge entlang und
eine Rückfluidhülse (107), um expandiertes Gas aus dem Instrument (100) abzulassen;
einen Kühlabschnitt, der mit dem Handhabungsabschnitt (110) verbunden ist, vom Handhabungsabschnitt (110) zur Spitze (103) verläuft und Folgendes aufweist:
den Wendelteil, wobei der Wendelteil eine Wendelanordnung hat, die sich um die Längsachse windet, und
eine Barriere, die nur im Kühlabschnitt angeordnet ist und sich um die Kryogenzuleitung (102) windet, wobei die Barriere einen gewundenen Kanal ergibt, der entlang der Innenseite der Außenfläche (101) am Umfang angeordnet ist, der von der Kryogenzuleitung (102) und der Innenseite der Außenfläche (101) des Instruments umgrenzt ist und die Zuleitung mit der Rückgashülse (107) verbindet, wobei die Barriere aus der Öffnung austretendes Kryogen den Kühlabschnitt kühlen lässt.

12. Kryochirurgisches Instrument (100) nach Anspruch 3, der Folgendes aufweist:
einen Kern, der nur im Wendelteil angeordnet ist und der vom Handhabungsabschnitt bis im Wesentlichen nahe an die Spitze (103) verläuft, wobei
die Kryogenzuleitung (102) sich um den Kern windet und wobei
die Windungen der Kryogenzuleitung (102) einen gewundenen Kanal ergeben, der entlang der Innenseite der Außenfläche (101) am Umfang angeordnet ist, der von der Kryogenzuleitung (102) und der Innenseite der Außenfläche (101) des Instruments (100) umgrenzt ist und der die Zuleitung (102) mit der Rückfluidhülse verbindet, wobei der gewundene Kanal aus der Öffnung austretendes Kryogen den Wendelteil kühlen lässt.

## Revendications

1. Instrument de cryochirurgie (100) qui est configuré de manière sélective pour être positionné dans un patient par rotation, comportant :
une section de manipulation (110) qui permet à un utilisateur de faire tourner l'instrument ; et
une partie d'alimentation en substance cryogénique,
une section de positionnement ayant un bout pointu au niveau d'une extrémité de celle-ci ; l'instrument de cryochirurgie étant **caractérisé par** :
une pluralité de lignes d'alimentation en substance cryogénique (102) qui fournit de la substance cryogénique à l'instrument (100) ; deux ou plusieurs échangeurs de chaleur discrets (106) disposés le long de chacune desdites une ou plusieurs lignes d'alimentation en substance cryogénique (102) et séparés par une longueur desdites une ou plusieurs lignes d'alimentation, dans lequel chaque échangeur de chaleur comporte des canaux qui s'enroulent en spirale autour de chaque ligne d'alimentation ;
une partie hélicoïdale (104), la partie hélicoïdale (104) de l'instrument de cryochirurgie (100) ayant une surface extérieure (101) en communication thermique avec les échangeurs de chaleur (106), dans lequel les échangeurs de chaleur (106) sont situés à l'intérieur de la partie hélicoïdale (104) de l'instrument de cryochirurgie (100), les échangeurs de chaleur (106) sont configurés pour recevoir de la substance cryogénique en provenance de la partie d'alimentation en substance cryogénique et pour permettre à la substance cryogénique reçue de refroidir la partie hélicoïdale (104) de l'instrument de cryochirurgie (100).

2. Instrument de cryochirurgie selon la revendication 1, dans lequel la partie hélicoïdale (104) est configurée pour
solliciter l'instrument de cryochirurgie (100) jusque dans le patient quand l'instrument (100) est tourné dans une première direction, par le biais de la section de manipulation (110), et
solliciter l'instrument de cryochirurgie (100) hors du patient quand l'instrument (100) est tourné dans une deuxième direction qui est opposée par rapport à la première, par le biais de la section de manipulation (110),
dans lequel l'instrument de cryochirurgie (100) est configuré pour avancer dans une direction le long d'un axe longitudinal de celui-ci quand l'instrument est tourné, par le biais de la section de manipulation (110), sans une majorité d'une force de positionnement qui est appliquée sur l'instrument de cryochirurgie (100) dans la direction.

3. Instrument de cryochirurgie (100) selon la revendication 1, dans lequel :
la surface extérieure (101) est une surface extérieure thermoconductrice sensiblement lisse (101) avec un côté intérieur, l'instrument de cryochirurgie (100) dans lequel :
au moins une partie de la section de manipulation (110) s'étend le long d'un axe longitudinal de l'instrument (100) et définit celui-ci, la section de manipulation ayant une isolation (108) le long au moins d'une partie d'une longueur de celle-ci, et
une gaine de fluide de retour (107) qui permet une évacuation de tout fluide détendu en provenance de l'instrument (100) ;
et chaque échangeur de chaleur (106) est en communication fluidique avec lesdites une ou plusieurs lignes d'alimentation en substance cryogénique (102), entoure lesdites une ou plusieurs lignes d'alimentation en substance cryogénique (102) dans la partie hélicoïdale (104), et transporte la substance cryogénique reçue jusqu'à un orifice au niveau d'une extrémité distale de celle-ci de manière proximale par rapport au bout (103), dans lequel chaque canal (109) est disposé de manière circonférentielle le long du côté intérieur de la surface extérieure (101) et qui interconnecte lesdites une ou plusieurs lignes d'alimentation à la gaine de gaz de retour (107), chaque échangeur de chaleur (106) permettant à la substance cryogénique en provenance desdites une ou plusieurs lignes d'alimentation de refroidir une section de refroidissement ; et l'instrument de cryochirurgie (100) comporte par ailleurs :
une voie de retour de fluide détendu entourant lesdites une ou plusieurs lignes d'alimentation en substance cryogénique (102) et liée par lesdites une ou plusieurs lignes d'alimentation en substance cryogénique (102) et le côté intérieur de la surface extérieure (101) de l'instrument (100) de telle sorte que le gaz dans la voie est en communication thermique avec le côté intérieur.

4. Instrument de cryochirurgie (100) selon la revendication 3, dans lequel la partie hélicoïdale (104) comprend de multiples spirales qui s'enroulent en spirale autour de l'axe longitudinal sur plus de 360 degrés.

5. Instrument de cryochirurgie (100) selon la revendication 3, dans lequel la partie hélicoïdale (104) est flexible et a une mémoire de forme.

6. Instrument de cryochirurgie (100) selon la revendication 6, dans lequel la partie hélicoïdale (104) est formée à partir de Nitinol, d'acier à ressort, ou d'un acier flexible.

7. Instrument de cryochirurgie (100) selon la revendication 1, dans lequel un taux de mise en spirale de la ligne d'alimentation en substance cryogénique (102) est constant.

8. Instrument de cryochirurgie (100) selon la revendication 3, dans lequel :
la partie hélicoïdale s'enroule en spirale autour de l'axe longitudinal selon un pas qui varie ; ou
un diamètre de la mise en spirale de la partie hélicoïdale varie.

9. Système (1100) comportant de multiples instruments de cryochirurgie des instruments de cryochirurgie (1101 ; 100) selon la revendication 3, dans lequel les instruments (1101 ; 100) sont regroupés ensemble le long de leurs longueurs.

10. Instrument de cryochirurgie (100) selon la revendication 3, dans lequel :
chaque ligne d'alimentation desdites une ou plusieurs lignes d'alimentation en substance cryogénique (102) transporte de la substance cryogénique jusqu'un ou plusieurs orifices le long d'une partie d'une longueur de celle-ci dans la partie hélicoïdale, au moins l'une des lignes d'alimentation a un orifice au niveau d'une extrémité distale de celle-ci de manière proximale par rapport au bout (103) ;
et la gaine de fluide de retour (107) entoure lesdites une ou plusieurs lignes d'alimentation en substance cryogénique (102) et est liée par lesdites une ou plusieurs lignes d'alimentation en substance cryogénique et le côté intérieur de la surface extérieure (101) de l'instrument (100) de telle sorte que le fluide dans la gaine est en communication thermique avec le côté intérieur, la gaine permettant à la substance cryogénique en provenance desdites une ou plusieurs lignes d'alimentation (102) de refroidir la partie hélicoïdale.

11. Instrument de cryochirurgie (100) selon la revendication 1, dans lequel la surface extérieure (101) est thermoconductrice et sensiblement lisse, et a un côté intérieur, l'instrument de cryochirurgie (100) comportant :
chaque ligne d'alimentation en substance cryogénique (102) qui transporte de la substance cryogénique jusqu'à un orifice au niveau d'une extrémité proximale par rapport au bout (103), dans lequel :
au moins une partie de la section de manipulation (110) s'étend le long d'un axe longitudinal de l'instrument, et définit celui-ci, et la section de manipulation a
une isolation (108) le long au moins d'une partie d'une longueur de celle-ci, et
une gaine de fluide de retour (107) permettant l'évacuation de tout gaz détendu en provenance de l'instrument (100) ;
une section de refroidissement connectée à la section de manipulation (110), s'étendant depuis la section de manipulation (110) jusqu'au bout (103), et comportant
la partie hélicoïdale, dans lequel la partie hélicoïdale a une configuration hélicoïdale s'enroulant en spirale autour de l'axe longitudinal, et
une barrière qui est disposée uniquement dans la section de refroidissement et qui s'enroule en spirale autour de la ligne d'alimentation en substance cryogénique (102), la barrière procurant un canal allant en spirale qui est disposé de manière circonférentielle le long du côté intérieur de la surface extérieure (101), qui est lié par la ligne d'alimentation en substance cryogénique (102) et le côté intérieur de la surface extérieure (101) de l'instrument, et qui interconnecte la ligne d'alimentation à la gaine de gaz de retour (107), la barrière permettant à la substance cryogénique de sortir de l'orifice pour refroidir la section de refroidissement.

12. Instrument de cryochirurgie (100) selon la revendication 3, comportant :
une partie centrale qui est disposée uniquement dans la partie hélicoïdale et qui s'étend depuis la section de manipulation jusqu'à sensiblement à proximité du bout (103), dans lequel
la ligne d'alimentation en substance cryogénique (102) s'enroule en spirale autour de la partie centrale, et dans lequel
la mise en spirale de la ligne d'alimentation en substance cryogénique (102) procure un canal de mise en spirale qui est disposé de manière circonférentielle le long du côté intérieur de la surface extérieure (101), qui est lié par la ligne d'alimentation en substance cryogénique (102) et le côté intérieur de la surface extérieure (101) de l'instrument (100), et qui interconnecte la ligne d'alimentation (102) à ladite une gaine de fluide de retour, le canal de mise en spirale permettant à la substance cryogénique de sortir de l'orifice pour refroidir la partie hélicoïdale.
